(19)

Europäisches
Patentamt
European
Patent Office
Office européen
des brevets

(11)  **EP 4 475 128 A1**

(12)  # EUROPEAN PATENT APPLICATION

(43) Date of publication:
**11.12.2024  Bulletin 2024/50**

(21) Application number: **23197981.6**

(22) Date of filing: **18.09.2023**

(51) International Patent Classification (IPC):
***G16B 40/00*** (2019.01)   ***G16H 50/20*** (2018.01)
***G16H 50/70*** (2018.01)

(52) Cooperative Patent Classification (CPC):
**G16B 40/00; G16H 50/20; G16H 50/70;**
G16H 50/80

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **08.06.2023  TW 112121368**

(71) Applicants:
 • **Acer Incorporated
   New Taipei City 221 (TW)**
 • **Chang Gung Memorial Hospital, Keelung
   Anle Dist. Keelung City 204201 (TW)**
 • **National Health Research Institutes
   Zhunan Town
   Miaoli County 350 (TW)**

(72) Inventors:
 • **TU, Chih-Wei
   221 New Taipei City (TW)**

 • **TSAI, Tsung-Hsien
   221 New Taipei City (TW)**
 • **CHAN, Yun-Hsuan
   221 New Taipei City (TW)**
 • **YANG, Ning-I
   204201 Keelung City (TW)**
 • **WU, I-Wen
   204201 Keelung City (TW)**
 • **YEH, Chi-Hsiao
   204201 Keelung City (TW)**
 • **LIAO, Yu-Chieh
   350 Miaoli County (TW)**
 • **TSAI, Ting-Fen
   350 Miaoli County (TW)**

(74) Representative: **2K Patentanwälte Blasberg
   Kewitz & Reichel
   Partnerschaft mbB
   Schumannstrasse 27
   60325 Frankfurt am Main (DE)**

(54)  **METHOD AND SYSTEM FOR ESTABLISHING DISEASE PREDICTION MODEL**

(57)   A method for establishing a disease prediction model is provided. The method includes the steps of extracting feature values for multiple microbiota features from microbiota data of each of a plurality of samples, selecting a portion of the extracted microbiota features as selected features, and training a disease prediction model. Each piece of training data used in training the disease prediction model includes (i) disease data for each of the samples and (ii) the feature values of the selected features for the sample. The microbiota features include species-level features, microbiota interaction features, and community-level features.

100

FIG. 1A

**(Cont. next page)**

**EP 4 475 128 A1**

FIG. 1B

**Description**

CROSS REFERENCE TO RELATED APPLICATIONS

This Application claims priority of Taiwan Patent Application No. 112121368, filed on June 08, 2023, the entirety of which is incorporated by reference herein.

**BACKGROUND OF THE INVENTION**

**Field of the Invention**

The present disclosure relates to feature engineering technology, in particular to a method and system for establishing a disease prediction model.

**Description of the Related Art**

The term "microbiota" refers to the collective population of microorganisms such as bacteria, viruses, fungi, and yeasts that reside within the human body. Together, they form a complex ecosystem that enables the proper functioning of the body. Similar to other ecosystems, the disruption of a single species within the microbiota can potentially impact the ecological balance. The most intricate microbiota within the human body is the gut microbiota, which has a significant influence on human health. The gut microbiota not only affects intestinal functions but also plays a crucial role in metabolism and the regulation of the immune system, thereby influencing overall health and internal equilibrium. Imbalances in the gut microbiota have been associated with various diseases, such as neurodegenerative disorders, cardiovascular diseases, diabetes, autoimmune diseases, cancer, etc. For instance, animal and human studies have indicated that "Fusobacterium nucleatum" may be a pathogenic factor in colorectal cancer. Experimental evidence has shown that reducing the occurrence of tumors is possible by eliminating Fusobacterium nucleatum through antibiotic treatments. However, due to the highly intricate nature of the gut microbiota, there is currently a lack of solutions for disease prediction based on the gut microbiota.

Therefore, there is a need for a method and system to establish a disease prediction model that can effectively utilize microbiota features to predict diseases. This disease prediction model can serve as an early warning system before the onset of diseases and provide further insights into their pathogenic mechanisms.

**BRIEF SUMMARY OF THE INVENTION**

An embodiment of the present disclosure provides a method executed by a computer system for establishing a disease prediction model. The method includes the steps of extracting feature values for multiple microbiota features from microbiota data of each of a plurality of samples, selecting a portion of the extracted microbiota features as selected features, and training a disease prediction model. Each piece of training data used in training the disease prediction model includes (i) disease data for each of the samples and (ii) the feature values of the selected features for the sample. The microbiota features include species-level features, microbiota interaction features, and community-level features.

In an embodiment, the species-level features include relative abundance data and presence/absence data of each of a plurality of species.

In an embodiment, the microbiota interaction features include the hierarchical ratio of two taxa on a taxonomic level.

In an embodiment, the community-level features include a Beta diversity matrix.

In an embodiment, the step of selecting a portion of the extracted microbiota features as the selected features includes the steps of inputting the disease data and the microbiota features into multiple feature selection models to obtain multiple feature pools, ranking the microbiota features based on the frequency of being selected into the feature pools by the feature selection models to obtain a feature ranking, and selecting a specified number of the microbiota features as the selected features based on the feature ranking. Each of the feature selection models selects one or more of the microbiota features to form the corresponding feature pool.

An embodiment of the present disclosure provides a system for establishing a disease prediction model. The system includes a storage device and a processing device. The storage device stores disease data and microbiota data of a plurality of samples. The processing device loads a program from the storage device to execute the steps of the aforementioned method.

The method and system provided by the present disclosure for establishing disease prediction models enhance the richness and representativeness of feature information by constructing microbiota features from multiple aspects. This not only improves the accuracy of disease prediction but also facilitates the interpretation of analysis results.

Furthermore, it helps deepen our understanding of the pathogenic mechanisms of diseases, aiding in the development of more effective treatment and prevention methods.

## BRIEF DESCRIPTION OF THE DRAWINGS

The present disclosure can be more fully understood by reading the subsequent detailed description and examples with references made to the accompanying drawings. Additionally, it should be appreciated that in the flow diagram of the present disclosure, the order of execution for each blocks can be changed, and/or some of the blocks can be changed, eliminated, or combined.

FIG. 1A is the flow diagram of a method for establishing a disease prediction model, according to an embodiment of the present disclosure;

FIG. 1B corresponds to the embodiment of FIG. 1A, and illustrates the schematic diagram of the method for establishing the disease prediction model;

FIG. 2 is the flow diagram of an embodiment of the feature selection stage;

FIG. 3 corresponds to the embodiment in FIG. 2, and illustrates the schematic diagram of multiple feature selection models and feature pools; and

FIG. 4 is the system block diagram of a system for establishing the disease prediction model, according to an embodiment of the present disclosure.

## DETAILED DESCRIPTION OF THE INVENTION

The following description provides embodiments of the invention, which are intended to describe the basic spirit of the invention, but is not intended to limit the invention. For the actual inventive content, reference must be made to the scope of the claims.

In each of the following embodiments, the same reference numbers represent identical or similar elements or components.

Ordinal terms used in the claims, such as "first," "second," "third," etc., are only for convenience of explanation, and do not imply any precedence relation between one another.

The descriptions provided for the embodiments of the devices or systems also apply to the embodiments of the methods, and vice versa.

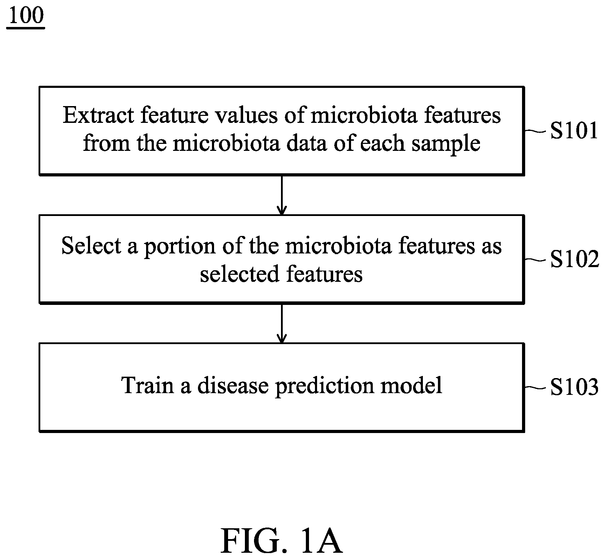

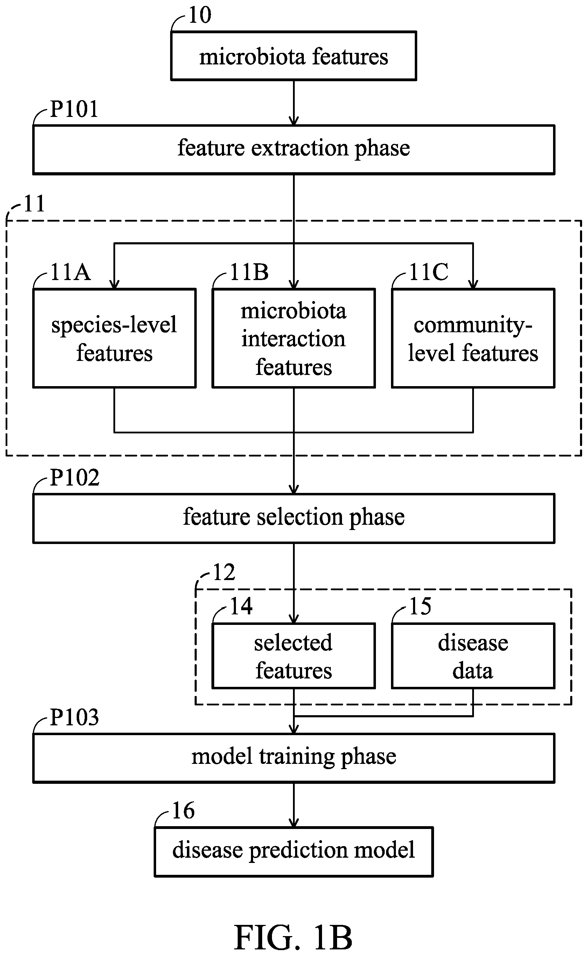

FIG. 1A is the flow diagram of a method 100 for establishing a disease prediction model, according to an embodiment of the present disclosure. As shown in FIG. 1A, the method 100 includes steps S101-S103. FIG. 1B corresponds to the embodiment of FIG. 1A and illustrates the schematic diagram of the method 100. As shown in FIG. 1B, the method 100 includes a feature extraction phase P101, a feature selection phase P102, and a model training phase P103. Please refer to both FIG. 1A and FIG. 1B for a better understanding of this embodiment.

Step S101 in FIG. 1A corresponds to the feature extraction phase P101 in FIG. 1B. In step S101 and the feature extraction phase P101, feature values of microbiota features 11 are extracted from the microbiota data 10 of each sample. Then, the method 100 proceeds to step S102.

As shown in FIG. 1B, the microbiota features 11 includes three types: species-level features 11A, microbiota interaction features 11B, and community-level features 11C. Each type includes one or more specific microbiota features.

Furthermore, in the embodiments of the present disclosure, the term "sample" refers to various specimens collected from the human body or animal body, such as blood, urine, saliva, feces, tissues, etc., which can be further analyzed, tested, or diagnosed. Each sample has corresponding microbiota data 10 and disease data 15, which describe the microbiota composition of the specimen and the disease type of the subject (i.e., the source of the specimen, whether it is a human or an animal). Since the gut microbiota is one of the most intricate microbiota in the human body and has a significant impact on human health, in the embodiments of the present disclosure, the gut microbiota is typically used as an example for the microbiota data 10, although the present disclosure is not limited thereto.

In the example of human gut microbiota, the microbiota data 10 can be collected through testing of fecal samples or collected through approaches such as gastrointestinal endoscopy. The disease data 15 can be collected using various medical diagnostic methods, such as blood tests, imaging examinations, and so on. However, the collection methods for microbiota data 10 and disease data 15 are not limited by the present disclosure. Other practices, such as blood testing, urine testing, or tissue biopsies, can also be used to collect relevant data. Additionally, the types of diseases that can be included in the disease data 15 are not limited by the present disclosure. Depending on the practical application and requirements, the disease types in the disease data 15 can include various conditions, such as infections, chronic diseases, cancers, etc. In the case of a perfectly healthy subject, the disease data 15 can indicate the absence of any disease. Alternatively, the disease data 15 can reflect the overall health status of the subject, such as the level of metabolic functioning or the quality of the immune system, although the present disclosure is not limited thereto.

In an embodiment, the microbiota data 10 can be represented by the quantity or abundance of various species in the sample. It is worth to note that the disclosure does not limit the microbiota data 10 to include all species present in the sample. In an embodiment, the inclusion of species in the microbiota data 10 can be determined based on their prevalence in the sample. For example, the microbiota data 10 may consider only species with a prevalence of over 10% in the sample. However, the selection of species in the microbiota data 10 is not limited by the present disclosure.

In an embodiment, the species-level features 11A provide more detailed information about each species, including relative abundance data and presence/absence data for each species.

Relative abundance data indicates the relative quantity of a species in the sample and is typically represented by the proportion of the species' DNA sequence in the sample. For example, if there are a total of 10 million bacterial cells in the sample, with 1 million of them being species A and 1.5 million being species B, the relative abundance value of species A would be 100/1000 = 0.1, and the relative abundance value of species B would be 150/1000 = 0.15. Therefore, the relative abundance value of 0.1 for species A and the relative abundance value of 0.15 for species B can be used as two feature values in the species-level features 11A.

Presence/absence data indicates whether a species is present or absent in the sample and is typically represented in binary form. For example, if species C is present in the sample and species D is absent, the presence/absence value of species C would be 1, and the presence/absence value of species D would be 0. Therefore, the presence/absence value of 1 for species C and the presence/absence value of 0 for species D can be used as two feature values in the species-level features 11A.

In an embodiment, the microbiota interaction features 11B include the hierarchical ratio between two taxa on a taxonomic level.

The hierarchical ratio is an indicator for describing the interaction relationship between two species. Typically, all species are classified based on the taxonomic hierarchy, including phylum, class, order, family, genus, and species. Then, the relative abundance ratios between two species on each level are calculated to describe the compositional structure of microbial communities in the sample. Suppose there are two taxa, Taxon A and Taxon B, on a specific level ($H_{Hierarchy}$). Taxon A consists of m species with relative abundances $X_1, X_2, X_3 ... X_m$, while Taxon B consists of n species with relative abundances $Y_1, Y_2, Y_3 ... Y_n$. The formula for calculating the hierarchical ratio is as follows:

$$\text{Hierarchical Ratio } (H_{Hierarchy}) = \frac{\sum_{i=1}^{m} X_i}{\sum_{j=1}^{n} Y_j}$$

The following Table 1 provides an example of microbiota data for Subject 01.

Table 1

| | Phylum | Class | ... | Species | Relative Abundance | Class Sum | Phylum Sum |
|---|---|---|---|---|---|---|---|
| Subject 01 | Phylum A | Class A | ... | a | 0.1 | 0.2 | 0.3 |
| | | | ... | b | 0.1 | | |
| | | Class B | ... | c | 0.1 | 0.1 | |
| | Phylum B | Class C | ... | d | 0.1 | 0.7 | 0.7 |
| | | | ... | e | 0.1 | | |
| | | | ... | f | 0.2 | | |
| | | | ... | g | 0.3 | | |

As shown in the microbiota data of Table 1, Phylum A includes three species: a, b, and c, with a relative abundance of 0.1 for each. Species a and b belong to Class A within Phylum A, while species c belongs to Class B within Phylum A. Phylum B includes four species: d, e, f, and g, with relative abundances of 0.1, 0.1, 0.2, and 0.3, respectively. All four species belong to Class C within Phylum B. The aforementioned "taxonomic level" can refer to any of the following: phylum, class, order, family, or genus. The microbiota interaction features (11B) may include hierarchical ratios obtained from one or more taxonomic levels. The calculation of hierarchical ratios will be demonstrated using the examples of "phylum" and "class" as the taxonomic levels.

When considering the taxonomic level of "phylum," the hierarchical ratio of Phylum A relative to Phylum B is calculated as the ratio between the sum of Phylum A's abundances and the sum of Phylum B's abundances in Table 1. The specific calculation is as follows:

$$\text{Hierarchical Ratio } \left(H_{\text{phylum}}\right) = \frac{\sum_{i=1}^{m} PhylumA_i}{\sum_{j=1}^{n} PhylumB_j} = \frac{(0.1 + 0.1 + 0.1)}{(0.1 + 0.1 + 0.2 + 0.3)} = \frac{0.3}{0.7}$$

When considering the taxonomic level of "class," the hierarchical ratios between Class A, Class B, and Class C are calculated based on the sum of each class's abundances in Table 1. The specific calculation is as follows:

$$\text{Hierarchical Ratio } (H_{\text{class}}) = \frac{\sum_{i=1}^{m} ClassA_i}{\sum_{j=1}^{n} ClassB_j} = \frac{(0.1 + 0.1)}{(0.1)} = \frac{0.2}{0.1}$$

$$\text{Hierarchical Ratio } (H_{\text{class}}) = \frac{\sum_{i=1}^{m} ClassB_i}{\sum_{j=1}^{n} ClassC_j} = \frac{(0.1)}{(0.1 + 0.1 + 0.2 + 0.3)} = \frac{0.1}{0.7}$$

$$\text{Hierarchical Ratio } (H_{\text{class}}) = \frac{\sum_{i=1}^{m} ClassA_i}{\sum_{j=1}^{n} ClassC_j} = \frac{(0.1 + 0.1)}{(0.1 + 0.1 + 0.2 + 0.3)} = \frac{0.2}{0.7}$$

The four calculated values: $\frac{0.3}{0.7}, \frac{0.2}{0.1}, \frac{0.1}{0.7}$, and $\frac{0.2}{0.7}$ can be used as four feature values for the microbiota interaction features 11B in the context of hierarchical ratios.

In an embodiment, community-level feature 11C includes a Beta Diversity Matrix. The Beta Diversity Matrix is a mathematical tool for comparing the similarity or dissimilarity between different microbial communities. Each element in the matrix represents the degree of difference between two microbial communities, typically measured using distance or similarity metrics.

In an embodiment, the Beta Diversity Matrix can utilize the Jaccard similarity as a measurement metric. Jaccard similarity is based on the presence/absence data described previously and calculates the ratio between the number of shared species and the total number of species present in two microbial communities. The value of Jaccard similarity ranges between 0 and 1. A value closer to 1 indicates a higher number of shared species and lower microbial dissimilarity between the samples. Conversely, a value closer to 0 indicates a lower number of shared species and higher microbial dissimilarity between the samples. In practice, the microbial data 10 of each sample can be used in rotation as a reference benchmark to calculate the microbial dissimilarity with other samples. Alternatively, pre-defined microbiota, such as the representative microbiota of diabetic patients, can be used as the reference benchmark. The calculation formula for Jaccard similarity is as follows:

$$J(X,Y) = \frac{|X \cap Y|}{|X \cup Y|} = \frac{|X \cap Y|}{|X| + |Y| - |X \cap Y|}$$

Here, $|X|$ and $|Y|$ represent the number of species in each community, $|X \cup Y|$ represents the total number of species combined in both microbial communities, and $|X \cap Y|$ represents the number of species shared between the communities.

The following Table 2 provides an example of disease categories and the presence/absence values of various species for Subjects 01-05.

Table 2

| Disease Category | Subject | Species A | Species B | Species C |
|---|---|---|---|---|
| 0 | Subject 01 | 0 | 1 | 1 |
| 0 | Subject 02 | 0 | 1 | 0 |
| 0 | Subject 03 | 1 | 1 | 1 |

(continued)

| Disease Category | Subject | Species A | Species B | Species C |
|---|---|---|---|---|
| 1 | Subject 04 | 0 | 0 | 0 |
| 1 | Subject 05 | 1 | 0 | 1 |

First, using Subject 01 as the reference benchmark, the calculation of Jaccard similarity between Subject 02 and Subject 01 is as follows:

$$J(X,Y) = \frac{|X \cap Y|}{|X \cup Y|} = \frac{|X \cap Y|}{|X| + |Y| - |X \cap Y|} = \frac{|Subject\ 01 \cap Subject\ 02|}{|Subject\ 01| + |Subject\ 02| - |Subject\ 01 \cap Subject\ 02|} = \frac{1}{2 + 1 - 1} = 0.5$$

Here, $|Subject\ 01|$ and $|Subject\ 02|$ represent the number of species present in the samples of Subject 01 and Subject 02, respectively. $|Subject\ 01 \cap Subject\ 02|$ represents the number of species shared by Subject 01 and Subject 02. According to Table 2, it can be observed that the sample of Subject 01 has two species (Species B and Species C), hence $|Subject\ 01| = 2$. The sample of Subject 02 only has one species (Species B), hence $|Subject\ 02| = 1$. The number of species shared by Subject 01 and Subject 02 is one (Species B), hence $|Subject\ 01 \cap Subject\ 02| = 1$.

Following the calculation described in the previous section, the Jaccard similarity values between all pairs of Subjects 01-05 can be calculated. These values form a Beta Diversity Matrix, which serves as the feature values for the community-level features 11C.

In another embodiment, the Beta Diversity Matrix can be measured using Bray-Curtis similarity as the metric. Bray-Curtis similarity is based on the relative abundance values and calculates the sum of absolute differences between the relative abundances of each species in two communities divided by the sum of their relative abundances. The resulting value ranges between 0 and 1. A value closer to 1 indicates lower microbial dissimilarity between the samples, while a value closer to 0 indicates greater microbial dissimilarity between the samples. The calculation formula for Bray-Curtis similarity is as follows:

$$BC_{ij} = 1 - \frac{\sum_{i=1}^{S} |M_{i1} - M_{i2}|}{\sum_{i=1}^{S} (M_{i1} + M_{i2})}$$

Here, $M_{i1}$ and $M_{i2}$ represent the relative abundance of the i-th species in the two samples, and S represents the total number of species.

Please refer back to FIG. 1A and FIG. 1B. In FIG. 1A, step S102 corresponds to the feature selection phase P102 in FIG. 1B. In step S102 and the feature selection phase P102, a portion of the microbiota features 11 are selected as selected features 14. Then, method 100 proceeds to step S103.

In an embodiment, in step S102 and the feature selection phase P102, a feature selection model is used to select the selected features 14. Various statistical models or machine learning models can be employed to implement the feature selection model. Examples of statistical models include the least absolute shrinkage and selection operator (Lasso) algorithm, stepwise logistic regression, statistical tests, etc. Machine learning models, on the other hand, can include decision trees, logistic regression, naive Bayes, random forest, support vector machines (SVM), fully connected neural networks, etc, but the present disclosure is not limited thereto.

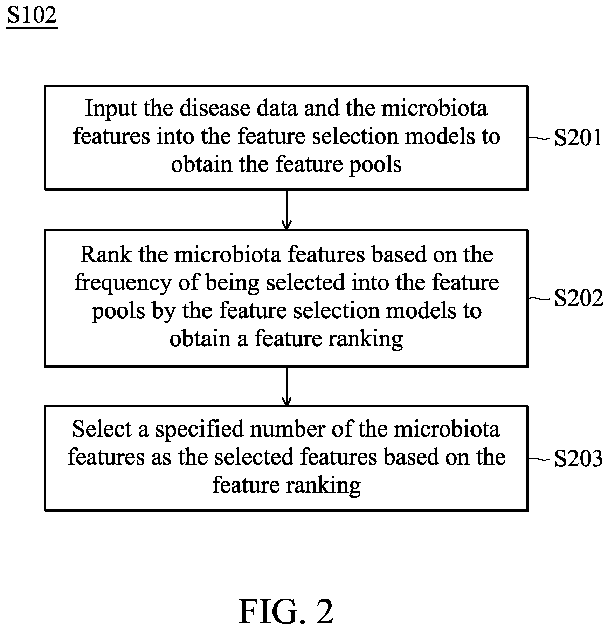

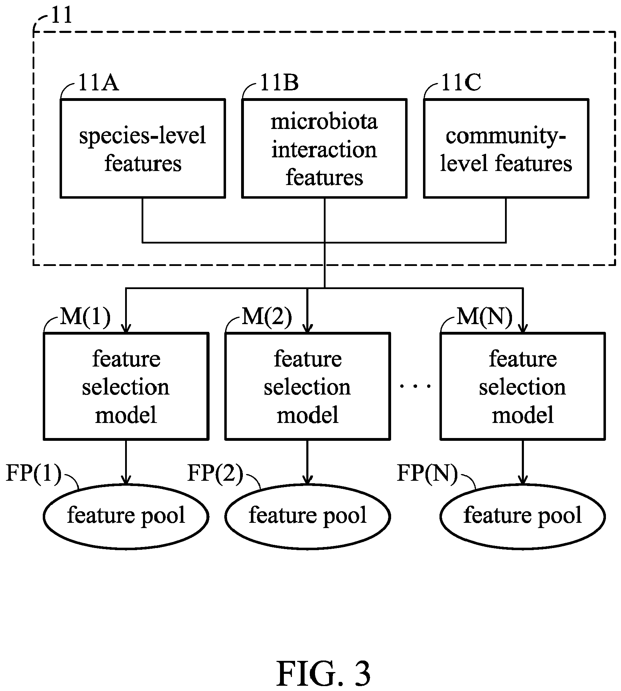

FIG. 2 is the flow diagram of one embodiment of step S102 from FIG. 1A. In this embodiment, multiple feature selection models are used for feature selection. As shown in FIG. 2, step S102 can further include steps S201-S203. FIG. 3 corresponds to the embodiment depicted in FIG. 2 and illustrates the schematic diagram of multiple feature selection models M(1)-M(N) and feature pools FP(1)-FP(N). Please refer to both FIG. 2 and FIG. 3 together for a better understanding of this embodiment.

In step S201, disease data 10 and the microbiota features including species-level features 11A, microbiota interaction features 11B, and community-level features 11C are input into N feature selection models, denoted as M(1)-M(N). This process generates N feature pools, denoted as FP(1)-FP(N). Then, the process proceeds to step S202.

As shown in FIG. 3, each of the feature selection models M(1)-M(N) selects one or more of the microbiota features including species-level features 11A, microbiota interaction features 11B, and community-level features 11C to form the corresponding feature pools. In FIG. 3, feature selection model M(1) outputs feature pool FP(1), feature selection model M(2) outputs feature pool FP(2), and so on. Different feature selection models M(1)-M(N) will select different features, resulting in varying microbiota features within feature pools FP(1)-FP(N).

Furthermore, the feature selection models M(1)-M(N) can be implemented using various statistical models or machine learning models. Examples of statistical models include the least absolute shrinkage and selection operator (Lasso) algorithm, stepwise logistic regression, statistical tests, etc. Machine learning models, on the other hand, can include decision trees, logistic regression, naive Bayes, random forest, support vector machines (SVM), fully connected neural networks, etc, but the present disclosure is not limited thereto.

In an embodiment, each of the feature selection models M(1)-M(N) calculates at least one statistical index for each microbiota feature one by one, and compares the statistical index with the corresponding critical value, so as to decide whether to select the microbiota feature to the corresponding feature pool. The critical value is a preset fixed value, which depends on the feature selection model itself. Statistical indicators can be, for example, P value, odds ratio, correlation coefficient, fold change, etc.

In an embodiment, the number of microbiota features to be selected into the corresponding feature pools FP(1)-FP(N) can be determined based on the accuracy of each feature selection model M(1)-M(N). For instance, the scree plot or elbow method can be employed to decide the number of microbiota features selected by the feature selection models. In the feature curve plot generated using the scree plot or elbow method, the horizontal axis typically represents the number of features used by the model, while the vertical axis represents a performance metric such as accuracy or area under the curve (AUC). The optimal number of features is determined by the changes in the performance metric. Conceptually, as the number of features increases, the performance metric tends to improve. However, there comes a point where the rate of improvement in the performance metric diminishes significantly. At this point, the number of features that achieves the best trade-off between performance and feature quantity can be selected.

Please refer back to FIG. 2. In step S202, the microbiota features are ranked based on the frequency of being selected into the feature pools FP(1)-FP(N) by the feature selection models M(1)-M(N). Thereby, a feature ranking is obtained. Then, the process proceeds to step S203.

The term "frequency of being selected into the feature pools FP(1)-FP(N) by the feature selection models M(1)-M(N)" can be understood as the count of feature pools in which a specific microbiota feature is included. A higher frequency indicates a higher importance or reference value for that microbiota feature. Therefore, microbiota features that rank higher in the feature ranking have greater importance or reference value.

In step S203, based on the feature ranking, a specified number of microbiota features are selected from the microbiota features 11 as the selected features. The specified number can be a pre-defined value or determined based on the accuracy of the feature selection models. Specifically, multiple test datasets can be used to obtain multiple accuracies of the feature selection models when selecting different numbers of microbiota features. The test datasets are solely used to evaluate the accuracy of the feature selection models. After obtaining the accuracies of the feature selection models for different numbers of microbiota features, one of the feature selection models is selected based on these accuracies. For instance, the feature selection model with the highest accuracy can be selected. Suppose 10 different numbers of microbiota features are selected to evaluate the accuracy of the feature selection models. Each feature selection model will obtain 10 accuracies, and with X feature selection models, there will be 10X accuracies. From these 10X accuracies, the highest accuracy can be identified to select the feature selection model with the highest accuracy. Then, based on the selected feature selection model's number and accuracy, the specified number can be determined using the previously mentioned methods such as the scree plot or elbow method.

Although the embodiment depicted in FIG. 3 shows that species-level features 11A, microbiota interaction features 11B, and community-level features 11C are collectively input into feature selection models M(1)-M(N) to obtain feature pools FP(1)-FP(N), but the present disclosure is not limited thereto. In another embodiment, species-level features 11A, microbiota interaction features 11B, and community-level features 11C can be individually input into feature selection models M(1)-M(N) to obtain N*3 feature pools. The microbiota features within these feature pools will be integrated to generate a feature set. Then, a similar approach to steps S202-S203 can be used to select, from the feature set, the features that respectively corresponds to the species-level features 11A, microbiota interaction features 11B, and community-level features 11C as the selected features 14.

Please refer to FIG. 1A and FIG. 1B. Step S103 in FIG. 1A corresponds to the model training phase P103 in FIG. 1B. In step S103 and the model training phase P103, a disease prediction model 16 is trained. The trained disease prediction model 16 can take the selected features of the subjects as input data and generate predictions about diseases. Depending on the purpose of disease prediction, the disease prediction model 16 can be a classification model that predicts disease types, or a regression model that predicts metabolic function indices, immune indices, or the probability of specific diseases, although the present disclosure is not limited thereto.

Furthermore, each training data 12 used for training the disease prediction model 16 includes (1) disease data 15 of each sample and (2) the feature values of the selected features 14 for that sample. During the model training phase P103, loss functions such as Mean Square Error (MSE), Mean Absolute Error (MAE), or cross-entropy can be used to compute the loss, which represents the difference between the predicted results of the disease prediction model 16 and the actual disease data 15. Moreover, an optimizer can be used to iteratively adjust the parameters of the disease prediction model 16 in order to minimize the loss and optimize the model. The optimizer can be implemented using algorithms such as Gradient Descent (GD), Stochastic Gradient Descent (SGD), or Adaptive Moment Estimation (Adam), but the present disclosure is not limited thereto. Taking the example of Gradient Descent optimizer, the gradients are computed by taking partial derivatives of the loss function, and then the parameters of the disease prediction model 16 are adjusted based on these gradients to reduce the loss. Through iterative feedback and parameter updates in the training process, the loss is gradually reduced until it converges to a minimum value.

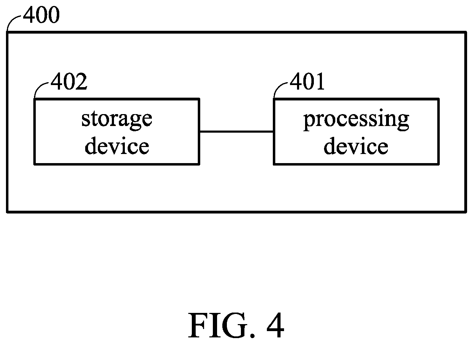

FIG. 4 is the block diagram of a system 400 for establishing the disease prediction model, according to an embodiment of the present disclosure. As shown in FIG. 4, the system 400 includes a processing device 401 and a storage device 402.

The system 400 can be a personal computer (such as a desktop or laptop computer) or a server computer running operating systems (such as Windows, Mac OS, Linux, UNIX, etc.) The system 400 can also be a mobile device such as a tablet or smartphone, although the present disclosure is not limited thereto.

The processing device 401 may include one or more hardware components for executing instructions, such as a central processing unit (CPU), a graphics processing unit (GPU), a microprocessor, a controller, a microcontroller, Application Specific Integrated Circuit (ASIC), Field Programmable Gate Array (FPGA), System on a Chip (SoC), etc., although the present disclosure is not limited thereto. In the embodiments of the present disclosure, the processing device 401 loads a program from the storage device 402 to execute steps S101 - S103 of the method 100 .

The storage device 402 can be any device with non-volatile memory (e.g., read-only memory (ROM), electrically-erasable programmable read-only memory (EEPROM), flash memory, or non-volatile random access memory (NVRAM)), such as hard disk drives (HDD), solid-state drives (SSD), or optical discs, although the present disclosure is not limited thereto. In the embodiments of the present disclosure, the storage device 402 stores a program, as well as the disease data and microbiota data of a plurality of samples. The program includes instructions for implementing the method 100. When processing device 401 loads the program from storage device 402, these instructions will be executed to implement the method 100.

The feature engineering approach provided by the present disclosure allows for the consideration of multi-level and multi-faceted microbiota features in disease prediction. Specifically, by establishing of species-level features 11A, the impact of microbial abundance and presence on diseases is taken into account, and the relationship between key pathogenic bacteria and diseases is highlighted, compared to existing methods. By establishing the microbiota interaction features 11B, the correlation between microbial growth and decline and diseases is taken into account. Additionally, by establishing microbiota features based on different taxonomic levels, it becomes possible to gain further insights into the functional units of microorganisms, even in the absence of knowledge about their functionality. This approach provides a starting point for subsequent analysis and research on pathogenic mechanisms. Furthermore, by establishing the community-level features 11C, the relationship between the disease and the "quality" and "quantity" of microbial communities, as well as relationship between the disease and the ecological balance between communities, are all taken into account.

In summary, the method and system provided by the present disclosure for establishing disease prediction models enhance the richness and representativeness of feature information by constructing microbiota features from multiple aspects. This not only improves the accuracy of disease prediction but also facilitates the interpretation of analysis results. Furthermore, it helps deepen our understanding of the pathogenic mechanisms of diseases, aiding in the development of more effective treatment and prevention methods.

The above paragraphs are described with multiple aspects. Obviously, the teachings of the specification may be performed in multiple ways. Any specific structure or function disclosed in examples is only a representative situation. According to the teachings of the specification, it should be noted by those skilled in the art that any aspect disclosed may be performed individually, or that more than two aspects could be combined and performed.

While the invention has been described by way of example and in terms of the preferred embodiments, it should be understood that the invention is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A method for establishing a disease prediction model, executed by a computer system, the method comprising:

   extracting feature values for multiple microbiota features from microbiota data of each of a plurality of samples;
   selecting a portion of the extracted microbiota features as selected features; and
   training a disease prediction model, wherein each piece of training data used in training the disease prediction model comprises (i) disease data for each of the samples and (ii) the feature values of the selected features for the sample;
wherein the microbiota features comprise species-level features, microbiota interaction features, and community-level features.

2. The method as claimed in claim 1, wherein the species-level features comprise relative abundance data and presence/absence data of each of a plurality of species.

3. The method as claimed in claim 1, wherein the microbiota interaction features comprise a hierarchical ratio between two taxa on a taxonomic level.

4. The method as claimed in claim 1, wherein the community-level features comprise a Beta diversity matrix.

5. The method as claimed in claim 1, wherein the step of selecting a portion of the extracted microbiota features as the selected features comprises:

    inputting the disease data and the microbiota features into multiple feature selection models to obtain multiple feature pools, wherein each of the feature selection models selects one or more of the microbiota features to form the corresponding feature pool;
    ranking the microbiota features based on frequency of being selected into the feature pools by the feature selection models to obtain a feature ranking; and
    selecting a specified number of the microbiota features are selected as the selected features based on the feature ranking.

6. A system for establishing a disease prediction model, comprising:

    a storage device, for storing disease data and microbiota data of a plurality of samples; and
    a processing device, loading a program from the storage device to execute the following steps:

        extracting feature values for multiple microbiota features from the microbiota data of each of a plurality of samples;
        selecting a portion of the extracted microbiota features as selected features; and
        training a disease prediction model, wherein each piece of training data used in training the disease prediction model comprises (i) the disease data for each of the samples and (ii) the feature values of the selected features for the sample;
        wherein the microbiota features comprise species-level features, microbiota interaction features, and community-level features.

7. The system as claimed in claim 6, wherein the species-level features comprise relative abundance data and presence/absence data of each of a plurality of species.

8. The system as claimed in claim 6, wherein the microbiota interaction features comprise a hierarchical ratio between two taxa on a taxonomic level.

9. The system as claimed in claim 6, wherein the community-level features comprise a Beta diversity matrix.

10. The system as claimed in claim 6, wherein the processing device further executes:

    inputting the disease data and the microbiota features into multiple feature selection models to obtain multiple feature pools, wherein each of the feature selection models selects one or more of the microbiota features to form the corresponding feature pool;
    ranking the microbiota features based on frequency of being selected into the feature pools by the feature selection models to obtain a feature ranking; and
    selecting a specified number of the microbiota features are selected as the selected features based on the feature ranking.

<u>100</u>

FIG. 1A

FIG. 1B

S102

```
┌─────────────────────────────────────────┐
│  Input the disease data and the microbiota │
│  features into the feature selection models to  │──S201
│           obtain the feature pools              │
└─────────────────────────────────────────┘
                      │
                      ▼
┌─────────────────────────────────────────┐
│   Rank the microbiota features based on the    │
│  frequency of being selected into the feature  │──S202
│     pools by the feature selection models to    │
│            obtain a feature ranking             │
└─────────────────────────────────────────┘
                      │
                      ▼
┌─────────────────────────────────────────┐
│   Select a specified number of the microbiota  │
│  features as the selected features based on the │──S203
│              feature ranking                    │
└─────────────────────────────────────────┘
```

FIG. 2

FIG. 3

400

402
storage
device

401
processing
device

# FIG. 4

**EUROPEAN SEARCH REPORT**

Europäisches
Patentamt
European
Patent Office
Office européen
des brevets

Application Number

EP 23 19 7981

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/244005 A1 (TEL HASHOMER MEDICAL RES INFRASTRUCTURE & SERVICES LTD [IL]) 24 November 2022 (2022-11-24) <br> * claim 1 * <br> * paragraph [0009] * <br> * paragraph [0011] * <br> * paragraph [0013] – paragraph [0014] * <br> * paragraph [0027] – paragraph [0028] * <br> * paragraph [0056] – paragraph [0058] * <br> * paragraph [0071] * <br> * paragraph [0076] * <br> * paragraph [0079] * <br> * paragraph [0096] – paragraph [0104] * <br> * paragraph [0108] * <br> * paragraph [0131] – paragraph [0153] * <br> * paragraph [0192] – paragraph [0193] * <br> * paragraph [0199] * <br> * paragraph [0205] – paragraph [0206] * <br> * paragraph [0223] – paragraph [0224] * <br> ----- | 1-10 | INV. <br> G16B40/00 <br> G16H50/20 <br> G16H50/70 |
| X | US 2021/355546 A1 (POORE GREGORY D [US] ET AL) 18 November 2021 (2021-11-18) <br> * claims 81-84,91-98 * <br> * paragraph [0007] – paragraph [0013] * <br> * paragraph [0040] – paragraph [0041] * <br> * paragraph [0119] – paragraph [0122] * <br> * paragraph [0126] – paragraph [0127] * <br> ----- <br> -/-- | 1-10 | TECHNICAL FIELDS SEARCHED (IPC) <br><br> G16B <br> G16H |

The present search report has been drawn up for all claims

| Place of search | Date of completion of the search | Examiner |
|---|---|---|
| Munich | 9 February 2024 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/128556 A1 (O'TOOLE PAUL [IE] ET AL) 28 April 2022 (2022-04-28)<br>* paragraph [0009] – paragraph [0010] *<br>* paragraph [0017] – paragraph [0028] *<br>* paragraph [0058] – paragraph [0061] *<br>* paragraph [0063] – paragraph [0065] *<br>* paragraph [0088] – paragraph [0090] *<br>* paragraph [0102] – paragraph [0103] *<br>* paragraph [0153] – paragraph [0156] *<br>* paragraph [0220] – paragraph [0222] *<br>* paragraph [0312] – paragraph [0319] *<br>----- | 1-10 | |
| A | WO 2019/036176 A1 (UBIOME INC [US]) 21 February 2019 (2019-02-21)<br>* claim 1 *<br>* paragraph [0051] *<br>* paragraph [0057] *<br>* paragraph [0063] *<br>----- | 1-10 | |

**TECHNICAL FIELDS SEARCHED      (IPC)**

The present search report has been drawn up for all claims

| Place of search | Date of completion of the search | Examiner |
|---|---|---|
| Munich | 9 February 2024 | Martínez Cebollada |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...........................................................................................
& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 23 19 7981

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2024

| Patent document cited in search report | | Publication date | Patent family member(s) | | Publication date |
|---|---|---|---|---|---|
| WO 2022244005 | A1 | 24-11-2022 | NONE | | |
| US 2021355546 | A1 | 18-11-2021 | AU | 2019372440 A1 | 27-05-2021 |
| | | | CA | 3118304 A1 | 07-05-2020 |
| | | | CN | 112930407 A | 08-06-2021 |
| | | | EP | 3874068 A1 | 08-09-2021 |
| | | | US | 2021355546 A1 | 18-11-2021 |
| | | | WO | 2020093040 A1 | 07-05-2020 |
| US 2022128556 | A1 | 28-04-2022 | AU | 2020255035 A1 | 28-10-2021 |
| | | | AU | 2020255277 A1 | 28-10-2021 |
| | | | CN | 114040986 A | 11-02-2022 |
| | | | CN | 114127317 A | 01-03-2022 |
| | | | EP | 3947743 A1 | 09-02-2022 |
| | | | EP | 3947744 A1 | 09-02-2022 |
| | | | JP | 2022527653 A | 02-06-2022 |
| | | | JP | 2022528466 A | 10-06-2022 |
| | | | KR | 20210149127 A | 08-12-2021 |
| | | | KR | 20220004069 A | 11-01-2022 |
| | | | TW | 202102683 A | 16-01-2021 |
| | | | TW | 202102684 A | 16-01-2021 |
| | | | US | 2022128556 A1 | 28-04-2022 |
| | | | US | 2022165361 A1 | 26-05-2022 |
| | | | WO | 2020201457 A1 | 08-10-2020 |
| | | | WO | 2020201458 A1 | 08-10-2020 |
| WO 2019036176 | A1 | 21-02-2019 | AU | 2018318756 A1 | 05-03-2020 |
| | | | CN | 111164706 A | 15-05-2020 |
| | | | EP | 3669377 A1 | 24-06-2020 |
| | | | JP | 7208223 B2 | 18-01-2023 |
| | | | JP | 2020530931 A | 29-10-2020 |
| | | | KR | 20200054203 A | 19-05-2020 |
| | | | SG | 11202001335U A | 30-03-2020 |
| | | | US | 2019050534 A1 | 14-02-2019 |
| | | | WO | 2019036176 A1 | 21-02-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- TW 112121368